# EUROPÄISCHE PATENTSCHRIFT

(11) **EP 0 427 956 B1**
(45) Veröffentlichungstag und Bekanntmachung des Hinweises auf die Patenterteilung: **18.01.1995**
(21) Anmeldenummer: 90119318.5
(22) Anmeldetag: 09.10.1990
(51) Int. Cl.: A61B 17/22, G10K 15/06

(54) **Verbesserung des Zündverhaltens bei Unterwasser-Funkenstrecken**
Improvement of the ignition characteristics of underwater spark gaps
Amélioration des charactéristiques d'allumage d'un éclateur en milieu aqueux

(30) Priorität: 15.11.1989 DE 3937904
(43) Veröffentlichungstag der Anmeldung: 22.05.1991
(73) Patentinhaber: DORNIER MEDIZINTECHNIK GMBH, D-82101 Germering (DE)
(72) Erfinder: Einars, Wolfram, Dr., W-8011 Neukeferloh (DE); Eizenhöfer, Harald, Dipl.-Phys., W-8000 München 19 (DE); Schutheiss, Reiner, Dr., W-8088 Eching (DE)
(74) Vertreter: Landsmann, Ralf, Dipl.-Ing.

(56) Entgegenhaltungen:
- EP-A- 0 296 912
- DE-A- 3 637 326
- US-A- 3 559 435

## Beschreibung

Die Erfindung betrifft ein Verfahren zur Verbesserung des Zündverhaltens an Funkenstrecken zur Erzeugung von Stoßwellen für die berührungsfreie Zerstörung von Konkrementen in Körpern von Lebewesen.

Stosswellenquellen finden in verschiedenen medizinischen und technischen Einrichtungen Verwendung. Funkenstrecken zur Stosswellenerzeugung haben sich beispielsweise bei der berührungsfreien medizinischen Stosswellenlithotripsie bewährt. Dabei wird durch elektrische Unterwasserfunkenentladung in einem Kondensator gespeicherte Energie in mechanische Stosswellenenergie umgewandelt. Diese Stosswellenenergie dient der berührungsfreien Zerttümmetung von Konkrementen in Körpern von Lebewesen. Die Konkremente werden in abgangsfähige Bruchstücke zerkleinert. Die Stosswelle wird in einem Brennpunkt eines Rotationsellipsoids erzeugt und im anderen Brennpunkt fokussiert. In diesem zweiten Brennpunkt befinden sich die zu zertrümmernden Konkremente.

Aus der DE-PS 23 51 247 und der DE-PS 26 35 635 sind verschiedene Ausführungsformen für Funkenstrecken bekannt.
Bei einer Funkenentladung in einer Funkenstrecke wird der Weg des Funkens durch den noch nahezu stromlosen Weg des Leaders bestimmt. Als Leader bezeichnet man hier einen Kanal zwischen den beiden Elektroden der Funkenstrecke, der zunächst beim Anliegen der Hochspannung entsteht und durch den dann der Weg des Funkens bestimmt wird. Der Leader folgt im wesentlichen dem Feldgradienten zwischen positiver und negativer Elektrode, doch bestimmen lokale Schwankungen des Feldgradienten maßgeblich den aktuellen Verlauf des Leaders.
Die bei Vorrichtungen zur Zerstörung von Konkrementen mittels Stoßwellen heute üblichen elektrischen Feldstärken zwischen den Elektrodenspitzen können zu thermischen Durchbrüchen führen, die charakterisiert sind durch lange Zündverzugszeiten im Bereich von 1 »s bis 1 ms, abhängig von Spannung, Leitfähigkeit, Elektrodenabstand und Geometrie, und durch große zeitliche Streuung, bedingt durch das stochastische Vorwachsen des Leaders.

Der Erfindung liegt die Aufgabe zugrunde, ein Verfahren anzugeben, mit dem der Zeit- und Energieaufwand zur Bildung des Durchschlagkanals einer Funkenstrecke verringert und dadurch geringere Streuung und längere Betriebsdauer ermöglicht wird.

Diese Aufgabe wird durch ein Verfahren nach Anspruch 1 gelöst. Ausgestaltungen sind Gegenstand von Unteransprüchen.

Eine Unterwasser-Funkenentladung erzeugt Stosswellen, die die Elektrodenspitzen erodieren, wodurch der Abstand der Elektrodenspitzen zunimmt und mit zunehmendem Abstand auch die Feldstärke beim Anlegen einer konstanten Hochspannung sinkt. In der Zündverzugszeit zwischen Anlegen der Hochspannung und deren Zusammenbruch und Freisetzung der im Kondensator gespeicherten Energie, fließt bereits Ladung vom Kondensator ab, was zu einer Verringerung der beim Durchbruch noch verfügbaren Energie führt. Je kürzer die Zündverzugszeit ist, desto größer bleibt die im Kondensator zur Verfügung stehende Energie. Während der Zündverzugszeit muß eine gewisse Stromdichte zum Aufbau des späteren Funkenkanals für die Hochspannungsentladung bis zum thermischen Durchbruch aufrechterhalten werden. Die Stromdichteverteilung ist bei homogener, ortsunabhängiger Leitfähigkeit direkt proportional zur Feldstärkeverteilung. Die Feldstärkeverteilung ihrerseits ist bestimmt durch die Geometrie aller beteiligten leitenden und nichtleitenden Werkstoffe und der anliegenden Spannung. Vorteilhaft ist, die Stromdichteverteilung auf einen engen Kanal zu begrenzen, der dann den zum Durchbruch führenden Leader enthält. Dies kann geschehen, indem man die Feldstärke oder die Leitfähigkeit oder beide auf der Achse und im achsnahen Raum zwischen den Elektrodenspitzen erhöht.

Erfindungsgemäß wird die Stromdichteverteilung dadurch beeinflußt, daß die Leitfähigkeit lokal im Bereich der Spitzen über ihre Temperaturverteilung heraufgesetzt wird. Eine Temperaturerhöhung im axialen Spitzenbereich wird herbeigeführt, indem durch das Fließen eines permanenten oder getakteten Stromes eine lokale Aufheizung erfolgt. Weiterhin führt der Strom zur Hydrolyse, wodurch an den Elektrodenoberflächen kleine Gasbläschen entstehen, die die Leaderbildung begünstigen.

An eine Unterwasser-Funkenstrecke wird eine Gleich- oder Wechselspannung angelegt, die zu Strömen im Bereich von 10 bis 100 mA führt. Damit wird der Funkenstrecke eine permanente Stromdichte aufgeprägt. Der zwischen den Elektroden fließende elektrolytische Strom führt zu den Effekten, daß sich die Wasser-Dipole in diesem Bereich ausrichten und auf den Elektrodenoberflächen eine Elektrolyse stattfindet.
Der Energie- und Zeitaufwand, der zum Aufbau eines leitfähigen Plasmakanals nötig ist, wird reduziert. Zum Zeitpunkt der Entladung ist mehr Ladung auf dem Kondensator vorhanden, wodurch die entstehende Stoßwelle eine höhere Intensität erhält. Die relative zeitliche Streuung der Zündverzugszeiten nimmt ab, dadurch haben die erzeugten Stoßwellen eine gleichmäßigere Intensität und eine geringere Amplitudenstreuung. Durch die Einengung des Kanals auf den axialen Bereich um den ersten Brennpunkt werden auch die Abbildungsfehler der im zweiten Brennpunkt fokussierten Stoßwelle kleiner, so daß dies zu einer Verringerung der statistischen Streuung der Stoßwellenintensitäten im therapeutischen Brennpunkt führt.

Beispielsweise wird bei einem Wechselstrom von 30 mA, einem Elektrodenabstand von 2,4 mm und einer Spannung von 14 kV (bei einem Kondensator von 80 nF) die Zündvertugszeit von 130 »s auf 30 »s reduziert. Die beim Durchzünden noch verfügbare Spannung ist dadurch erst auf 90 % der ursprünglichen Spannung abgefallen anstatt auf ca. 30 % bei bekannten Funkenstrecken. Die Elektroden können deutlich länger betrieben werden und zünden zuverlässiger auch bei geringen Spannungen.
Es ist aber auch möglich, den Strom gepulst korreliert mit der Hauptentladung zu schalten, so daß nur dann ein Strom fließt, wenn eine Entladung des Kondensators unmittelbar folgt.

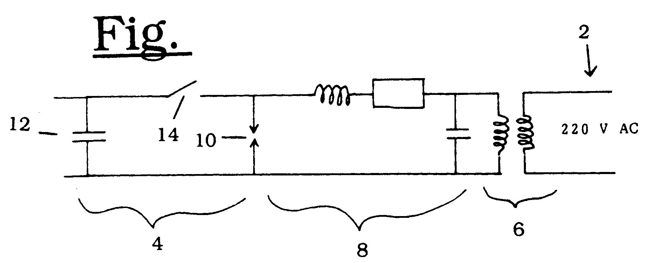

Die Erfindung wird anhand einer Figur näher erläutert.
Die Figur zeigt das Prinzipschaltbild der Erfindung
Die Figur zeigt ein Prinzipschaltbild einer Entladungsschaltung für Vorrichtungen zur Zertümmerung von Konkrementen. Zwischen der 220 V Wechselspannungsseite 2 und dem Stoßwellenkreis 4 ist ein Transformator 6 und eine Strombegrenzungsschaltung 8 zum Schutz vor transienten Hochspannungs-Pulsen aus der Hauptentladung vorgesehen. Die Strombegrenzungsschaltung 8 läßt über die Funkenstrecke 10 einen Strom 10 permanent fließen, wodurch die Zündverzugszeiten zum Durchschlagen der Funkenstrecke 10 bei Entladung des Kondensators 12 durch Schließen des Schalters 14 verkürzt wird. Auch eine Versorgung über ein Schaltnetzteil oder eine Gleichspannungsquelle, beispielsweise eine Batterie, ist möglich.

## Patentansprüche

1. Verfahren zur Verbesserung des Zündverhaltens an Funkenstrecken mit zwei sich gegenüberliegenden Elektroden zur Erzeugung von Stoßwellen für die berührungslose Zerstörung von Konkrementen in Körpern von Lebewesen, **dadurch gekennzeichnet**, daß an der Funkenstrecke zwischen den Elektroden eine Spannung angelegt wird, die sehr viel kleiner ist als die Durchschlagsspannung und einen kleinen elektrischen Strom zwischen den Elektroden bewirkt.

2. Verfahren nach Anspruch 1, **dadurch gekennzeichnet**, daß die Spannung permanent an der Funkenstrecke angelegt wird.

3. Verfahren nach Anspruch 1, **dadurch gekennzeichnet**, daß die Spannung gepulst korreliert mit der Hauptentladung angelegt wird.

4. Verfahren nach einem der vorherigen Ansprüche, **dadurch gekennzeichnet**, daß ein zur Anlegung der kleinen Spannung vorgesehener Versorgungsteil vor bei der Unterwasser-Funkenentladung entstehenden transienten Hochspannungspulsen geschützt wird.

5. Verfahren nach einem der vorherigen Ansprüche, **dadurch gekennzeichnet**, daß zwischen den Elektroden eine Gleichspannung angelegt wird.

6. Verfahren nach einem der Ansprüche 1 bis 4, **dadurch gekennzeichnet**, daß zwischen den Elektroden eine Wechselspannung angelegt wird.

7. Schaltung an Funkenstrecken mit zwei sich gegenüberliegenden Elektroden zur Erzeugung von Stoßwellen für die berührungslose Zerstörung von Konkrementen in Körpern von Lebewesen, **dadurch gekennzeichnet**, daß Mittel zur Verbesserung des Zündverhaltens vorgesehen sind, die an der Funkenstrecke zwischen den Elektroden eine Spannung anlegen, die sehr viel kleiner als die DurchschIagsspannung ist und die einen kleinen elektrischen Strom zwischen den Elektroden fließen läßt.

## Claims

1. Process for improving the ignition behaviour in spark paths with two oppositely placed electrodes for generating shockwaves for contact-free destruction of concrements in bodies of living creatures, **characterised in that** a voltage, which is very much lower than the breakdown voltage and generates a small electrical current between the electrodes, is applied to the spark path between the electrodes.

2. Process according to claim 1, **characterised in that** the voltage is permanently applied to the spark path.

3. Process according to claim 1, **characterised in that** the voltage is pulsed and correlated with the main discharge.

4. Process according to one of the above claims, **characterised in that** a delivery element provided for application of the low voltage is protected from transient high-voltage pulses which develop during an underwater spark discharge.

5. Process according to one of the above claims, **characterised in that** a direct-current voltage is applied between the electrodes.

6. Process according to one of claims 1 to 4, **characterised in that** an alternating-current voltage is applied between the electrodes.

7. Circuit in spark paths with two oppositely placed electrodes for generating shockwaves for contact-free destruction of concrements in the bodies of living creatures, **characterised in that** means for improving the ignition behaviour are provided, which apply a voltage to the spark path between the electrodes, which is very much lower than the breakdown voltage and which allows a low electrical current to flow between the electrodes.

## Revendications

1. Procédé pour améliorer les caractéristiques d'allumage d'éclateurs comportant deux électrodes disposées en vis-à-vis destinés à la production d'ondes de choc pour détruire sans contact des concrétions dans le corps d'êtres vivants, caractérisé par le fait que l'on applique à l'éclateur, entre les électrodes de celui-ci, une tension qui est beaucoup plus faible que la tension de claquage et engendre un courant électrique de faible valeur entre lesdites électrodes.

2. Procédé selon la revendication 1, caractérisé par le fait que la tension est appliquée de manière permanente à l'éclateur.

3. Procédé selon la revendication 1, caractérisé par le fait que la tension est appliquée de manière pulsatoire en corrélation avec la décharge principale.

4. Procédé selon l'une des revendications précédentes, caractérisé par le fait qu'une partie d'alimentation prévue pour l'application de la tension de faible valeur est protégée contre les impulsions transitoires à haute tension qui apparaissent lors de la décharge d'étincelles en milieu aqueux.

5. Procédé selon l'une des revendications précédentes, caractérisé par le fait que l'on applique une tension continue entre les électrodes.

6. Procédé selon l'une des revendications 1 à 4, caractérisé par le fait que l'on applique une tension alternative entre les électrodes.

7. Circuit pour éclateurs avec deux électrodes disposées en vis-à-vis destiné à la production d'ondes de choc pour détruire sans contact des concrétions dans le corps d'êtres vivants, caractérisé par le fait qu'il est prévu des moyens pour améliorer les caractéristiques d'allumage, lesquels moyens appliquent entre les électrodes de l'éclateur une tension qui est très inférieure à la tension de claquage et laissent passer un courant électrique de faible valeur entre lesdites électrodes.
